# EUROPÄISCHE PATENTSCHRIFT

(11) **EP 1 781 206 B1**
(45) Veröffentlichungstag und Bekanntmachung des Hinweises auf die Patenterteilung: **04.03.2015**
(21) Anmeldenummer: 05766106.8
(22) Anmeldetag: 20.07.2005
(51) Int. Cl.: A61F 2/06, D05B 3/00, D05B 23/00, D05B 21/00

(54) **VERFAHREN ZUR HERSTELLUNG EINER TEXTILEN GEFÄSSPROTHESE MIT EINER LÄNGSBIEGUNG**
METHOD OF MANUFACTURE OF A LONGITUDINALLY FLEXIBLE TEXTILE VASCULAR PROSTHESIS
PROCÉDÉ DE FABRICATION D'UNE PROTHESE VASCULAIRE TEXTILE A FLEXION LONGITUDINALE

(30) Priorität: 21.07.2004 DE 102004035272; 12.08.2004 DE 102004039980
(43) Veröffentlichungstag der Anmeldung: 09.05.2007
(60) Teilanmeldung: 11169560.7
(73) Patentinhaber: Aesculap AG, 78532 Tuttlingen (DE); Sievers, Hans-Hinrich, Prof. Dr. med., 24119 Kronshagen (DE)
(72) Erfinder: SIEVERS, Hans-Hinrich, 24119 Kronshagen (DE); LIPPOTH, Lisa, 78588 Denkingen (DE); MERCKLE, Christof, 68199 Mannheim (DE); GOLDMANN, Helmut, 78532 Tuttlingen (DE); SCHERBERICH, Jürgen, 47906 Kempen (DE)
(74) Vertreter: Patentanwälte Ruff, Wilhelm, Beier, Dauster & Partner
(86) Internationale Anmeldenummer: PCT/EP2005/007907
(87) Internationale Veröffentlichungsnummer: WO 2006/008148

(56) Entgegenhaltungen:
- EP-A- 0 251 331
- EP-A- 1 178 144
- WO-A-00/47271
- DE-A1- 10 162 821
- DE-A1- 10 242 153
- GB-A- 2 355 728
- US-A- 2 836 181
- US-A- 4 169 422
- US-A- 4 544 599
- US-A- 4 730 566
- US-A1- 2003 088 305

## Beschreibung

Die Erfindung betrifft ein Verfahren zur Erzeugung einer gezielten, bleibenden Krümmung an einer künstlichen Gefäßprothese.

Aus der WO 03/034948 A1 ist eine plissierte Gefäßprothese bekannt, bei der eine Bogenform dadurch stabilisiert wird, dass jeweils zwei benachbarte Faltenberge der Plissierung durch querverlaufende Nähte miteinander vernäht werden, wobei sich die querverlaufenden Nähte in etwa über den halben Durchmesser der Gefäßprothese in Querrichtung erstrecken. Es ist unter anderem aus dieser Druckschrift auch bekannt, die Bogenform von Prothesen durch im Bereich des Bogens eingelegte Stents zu stabilisieren. In der WO 03/051232 A1 sind zur Stabilisierung des Bogens von Gefäßprothesen verschiedene Kombinationen von Längs- und/oder Quernähten beschrieben. Die Ergebnisse sind zufriedenstellend. Eine einfachere Herstellungsweise, insbesondere eine maschinelle Ausbildung der Bogenstabilisierung ist jedoch erwünscht.

In einer nicht vorveröffentlichten älteren Patentanmeldung DE 103 28 175.4 wird unter anderem vorgeschlagen, die Plissierungsfalten in radialer oder tangentialer Richtung zusammenzudrücken, um maschinelle Nähte in einfacher Weise ausbilden zu können. Hierbei wird eine Verformung der Gefäßprothese im Querschnitt in Kauf genommen.

Die DE 102 42 153 A1 offenbart eine textile Gefäßprothese mit einer umlaufenden von Falten gebildeten Plissierung und einer mindestens über einen Teilabschnitt ausgebildeten bogenförmigen Längsbiegung. Durch eine längs des Teilabschnittes verlaufende Längsnaht erfolgt eine asymmetrische verkürzende Raffung der Prothesenwand.

Der Erfindung liegt die Aufgabe zugrunde, auf einfache Weise eine Stabilisierung des Bogens von Gefäßprothesen auszubilden, die insbesondere auch eine einfache maschinelle Fertigung mit verbessertem Ergebnis erlaubt.
Die Aufgabe wird gelöst durch ein Verfahren gemäß Anspruch 1.

Das Verfahren ist dadurch gekennzeichnet, dass eine Gefäßprothese, die gegebenenfalls einen thermisch vorfixierten Bogen aufweist, axial gestaucht bzw. kompaktiert wird und die Naht an der Stelle bzw. den Stellen, an denen eine elastische Ausdehnung verhindert werden soll, angebracht wird, wonach man die Prothese unter Bildung der Bogenform axial expandieren lässt.
Die gestauchte Prothese wird von einem Stützrohr aufgenommen, auf dem auch die Stauchung vorgenommen wird.

Dadurch, dass die Gefäßprothese auf ein Stützrohr aufgebracht und auf diesem gestaucht wird, ist der Nähvorgang besonders einfach. Die Gefäßprothese wird über ihre Länge sehr gleichmäßig gestaucht. Das Stützrohr verhindert eine Biegung der Gefäßprothese während des Nähvorgangs. Der Faden, der in axialer Richtung in die Wandung des elastischen Rohres eingenäht wird, verhindert nach Abziehen der Gefäßprothese vom Stützrohr, dass die Gefäßprothese auf einem Teil des Umfangs, nämlich im Bereich des genähten Bereichs zurückfedern kann. Auf der gegenüberliegenden Seite der Naht wird die Gefäßprothese weniger stark behindert zurückzufedern. Auf diese Weise nimmt die Gefäßprothese nach Abziehen von dem Stützrohr automatisch einen Bogen an, dessen Krümmung von der ursprünglichen Stauchung und der Länge der Naht abhängig ist.

Es ist ein Vorteil, wenn das Stützrohr während des Nähvorgangs in axialer Richtung bewegt wird. Dies ermöglicht eine gleichförmige Naht.

Es ist vorteilhaft, die Naht mittels einer Nähnadel zu erzeugen. In diesem Fall ist der einfache Kettenstich möglich und empfehlenswert.

Vorzugsweise taucht die Nähnadel durch die Gefäßprothese und durch ein in die Wandung des Stützrohres eingebrachtes Langloch oder eingebrachten Schlitz in das Innere des Stützrohres ein. Dann kann der Faden innerhalb der Gefäßprothese verknotet werden.

Aus dem gleichen Grund ist es vorteilhaft, wenn ein Fadengreifer im Inneren der Gefäßprothese und insbesondere des Stützrohres angebracht ist, der den Faden von der Nadel abnimmt und verkettet.

Um die Geschwindigkeit des Verfahrens zu erhöhen, ist es sinnvoll, den Fadengreifer rotieren zu lassen.

Es erhöht die Sicherheit, wenn das Stützrohr während seiner axialen Bewegung den rotierenden Fadengreifer umschließt. Es werden dadurch Verletzungen durch Hineingreifen vermieden.

Es ist von ganz besonderem Vorteil, wenn die Gefäßprothese vor der Aufnahme auf das Stützrohr auf links gewendet wird, also die Innenoberfläche nach außen und umgekehrt. Das bewirkt nämlich, dass die im Kettenstichverfahren innen vorgenommene Verknotung anschließend wieder nach außen gewendet wird. Damit stellen die Knoten kein Hindernis für die Blutzirkulation dar. Das so angewendete Verfahren erlaubt zudem eine genauere Kontrolle der Nähnaht und eine bessere Bearbeitungsmöglichkeit der Verriegelungen oder Verknotungen am Anfang und Ende der Naht. Beispielsweise kann das Kürzen der Endfäden wesentlich genauer durchgeführt werden.

Es ist vorteilhaft, wenn die Stauchung der Gefäßprothese auf dem Stützrohr manuell vorgenommen wird. Das ermöglicht die einfachste und schnellste Anwendung des Verfahrens.

Bei absoluten Präzisionsarbeiten oder mehrfach wiederkehrenden gleichen Arbeitsvorgängen kann es auch sinnvoll sein, eine Stauchungsvorrichtung für die Stauchung einzusetzen.

Dazu ist es sinnvoll, die Gefäßprothese wenigstens an einem verstellbaren Endanschlag abzustützen. Damit ist eine genaue Einstellmöglichkeit und Replizierbarkeit des Vorgangs gewährleistet.

Für eine Vorrichtung zur Herstellung einer gezielten bleibenden Krümmung einer Gefäßprothese ist es vorteilhaft, wenn ein Stützrohr vorhanden ist, auf das die Gefäßprothese in gestauchter Form geschoben werden kann. Für den anschließenden Nähvorgang ist dadurch ein sicherer Halt gewährleistet.

Vorzugsweise entspricht der Außendurchmesser des Stützrohres in etwa dem Innendurchmesser der Gefäßprothese. Dadurch wird der sichere Halt unterstützt.

Es ist von Vorteil, wenn das Stützrohr mit einer Vorschubeinrichtung verbunden ist. Dann wird die Gefäßprothese in gestauchter Form mit festem Sitz auf dem Stützrohr unterhalb der Nadel geführt, was zu einem gleichmäßigen Nahtbild führt.

Weitere Merkmale und Vorteile der Erfindung ergeben sich aus der nachfolgenden Beschreibung von bevorzugten Ausführungsformen in Verbindung mit der Zeichnung und den Unteransprüchen. Hierbei können die einzelnen Merkmale jeweils für sich alleine oder in Kombination miteinander verwirklicht sein. In der Zeichnung zeigen:
- Figur 1:: eine Seitenansicht eines erfindungsgemäß hergestellten Aortenbogens,
- Figur 2:: einen Schnitt entlang der Linien II.-II. nach Figur 1,
- Figur 3:: eine schematische Darstellung des Bereichs A an der Innenseite des Aortenbogens nach Figur 1,
- Figur 4:: eine schematische Darstellung des Bereichs A in einer anderen Ausführungsform,
- Figur 5:: eine schematische Darstellung des Bereichs A in einer weiteren Ausführungsform,
- Figur 6:: eine schematische Darstellung des Bereichs A in einer weiteren Ausführungsform,
- Fig. 7:: schematisch eine erfindungsgemäß hergestellte Gefäßprothese in perspektivischer Darstellung,

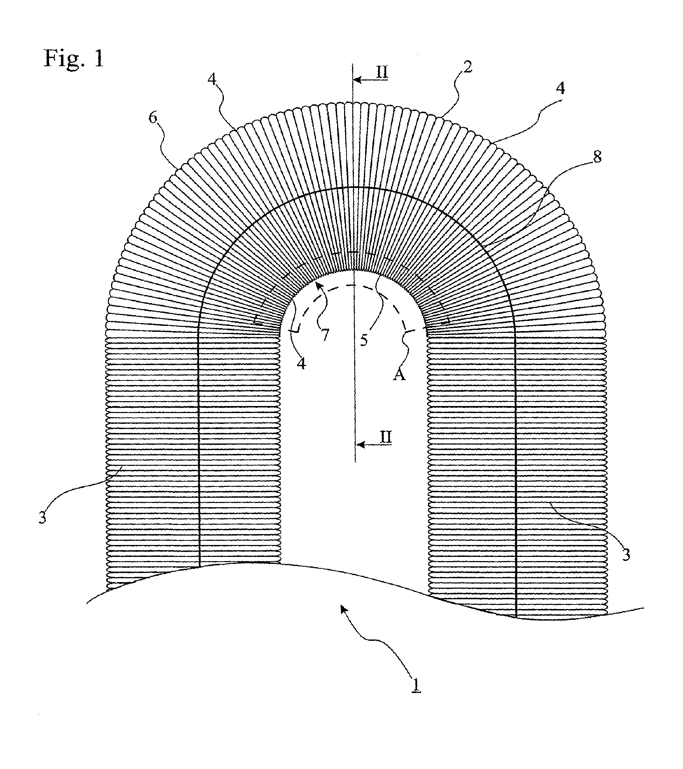

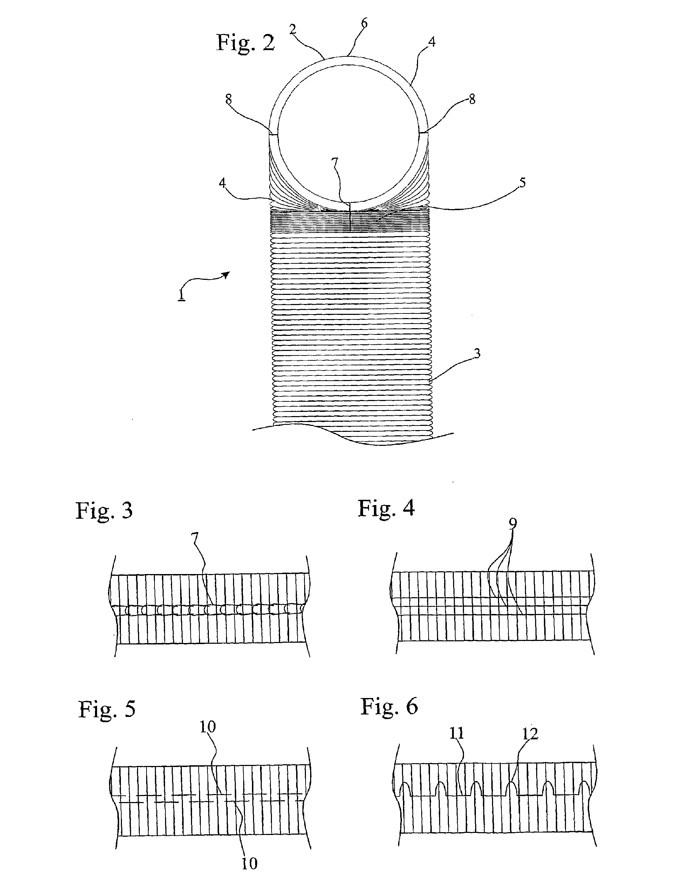

Bei der in der Zeichnung dargestellten Ausführungsform gemäß den Figuren 1 und 2 ist ein Aortenbogen 1 vorgesehen, der einen Bogenabschnitt 2 mit einer Umbiegung von 180° aufweist. An den Bogenabschnitt schließen sich gerade Endstücke 3 an, die zum Annähen an die verbleibenden Reste der natürlichen Aorta dienen. Der Aortenbogen 1 besitzt einen Innendurchmesser von 33 mm sowie eine Plissierung mit sieben Falten 4 pro cm. Die Faltentiefe beträgt ca. 1,5 mm. Die Falten 4 sind an der Bogeninnenseite 5 dicht aneinander liegend und erweitern sich stetig bis zur Bogenaußenseite 6. Der verdichtete kompakte Zustand der Falten an der Bogeninnenseite 5 wird durch eine einzige Längsnaht 7 stabilisiert. Die Stiche der Längsnaht durchdringen den kompaktierten Faltenbalg an der Bogeninnenseite in radialer Richtung und bilden zusammenhängende schlaufenartige Schlingen, die jeweils ca. drei Falten umschließen. Auf den beiden Seiten des Aortenbogens befindet sich je eine Guideline 8, wobei die beiden Guidelines verschieden breit ausgebildet sind. Das Nahtmaterial der Längsnaht 7 besitzt eine Färbung, die sich von der weißen Grundfärbung des Aortenbogens auch im mit Blut getränktem Zustand deutlich abhebt.

Der Faden der Längsnaht 7 ist ein multifiler mit Silikon beschichteter Faden mit der Fadenstärke 3/0. Die Naht beruht auf einem Einfadensystem, bei der ein Faden im Einfachkettenstich (Fig. 3) vernäht ist. Bei diesem System wird eine Fadenschlaufe radial durch die Prothesenwand geführt, in Nahtrichtung umgelegt und beim nächsten Stich durch die nächste Schlaufe erfasst. Dadurch bildet sich eine Längskette von zusammenhängenden Schlaufen. Auf der anderen Seite ist der einzelne Faden von Stich zu Stich in Längsrichtung geführt.

Zur Herstellung der Längsnaht wird zweckmäßigerweise ein zur Bildung des Aortenbogens verwendeter Abschnitt einer gewirkten Gefäßprothese mit Plissierung bzw. Faltenbalg auf einer Stützvorrichtung geradlinig in Längsrichtung so weit wie möglich zusammengeschoben, bis die einzelnen Falten des Faltenbalges in kompaktiertem Zustand parallel dicht nebeneinander liegen. In diesem Zustand wird die Längsnaht zum Beispiel mit einer Langarmnähmaschine angefertigt, wobei der Faden die kompaktierten Falten radial durchdringt. Die Fadenenden werden durch Fadenverdichtung gegen unerwünschtes Aufgehen verriegelt. Es sind auch Zwischenverriegelungen möglich. Beim Abziehen des kompaktierten Faltenbalges von der Stützvorrichtung entspannen sich die Falten der Plissierung wieder bis auf die Falten, die durch die Längsnaht an der Bogeninnenseite an einem Auseinandergehen gehindert sind. Dadurch bildet sich ein gleichmäßiger Aortenbogen aus. Die Länge der Längsnaht beträgt ca. 50 mm.

Die Figuren 3 bis 6 zeigen verschiedene Ausführungsformen von Längsnähten, die sich ausnahmslos an der Bogeninnenseite befinden. Figur 3 zeigt die einzige Längsnaht 7 der Ausführungsform nach Figur 1. Bei der Ausführungsform nach Figur 4 sind drei parallele Längsnähte 9 vorgesehen, die einen Abstand von 1 bis 2 mm voneinander haben. Figur 5 zeigt Teillängsnähte 10, die in zwei Reihen im Abstand von 2 mm unter gegenseitigem Versatz angeordnet sind. Die Teillängsnähte haben eine Länge von ca. 10 mm. Die Fadenenden sind jeweils gegen Aufgehen gesichert. Figur 6 zeigt in einer Linie verlaufende Teillängsnähte 11, die jeweils Unterbrechungen 12 zwischen sich aufweisen. Die Unterbrechungen 12 haben die Folge, dass der Faltenbalg in deren Bereich nicht kompaktiert ist, d.h. nach der Anfertigung der Naht sich wieder ausdehnt. Durch solche Unterbrechungen ist es möglich, die Größe der Bogenweite bzw. des Bogenradius zu steuern. Die Teillängsnähte 11 und/oder die Unterbrechungen 12 zwischen diesen können durch entsprechende Steuerung der Naht auch unterschiedliche Größe aufweisen, wodurch von der Kreisbogenform abweichende Bogenformen ausgebildet werden können. Ist es bei Nähten, die auf der einen Seite ein anderes Erscheinungsbild haben als auf der anderen Seite erwünscht, eine bestimmte Nahtseite außen zu haben, dann kann dies dadurch beeinflusst werden, dass die Prothese vor dem Zusammenschieben und Anbringen der Naht gegebenenfalls umgestülpt und danach wieder zurückgestülpt wird.

Durch das Zusammenhalten der kompaktierten Bereiche des Faltenbalgs an der Bogeninnenseite wird eine gleichmäßige Bogenform erhalten. Der kreisrunde Innenquerschnitt des Aortenbogens bleibt trotz der maschinellen Fertigung der Naht, bei der ein gewisser Anpressdruck erforderlich ist, aufrecht erhalten.

Der erfindungsgemäße Aortenbogen kann in üblicher Weise imprägniert und in steriler Form verpackt werden. Falls nicht die gesamte Bogenlänge zum Einsatz kommt, kann vor der Operation ein entsprechender Bogenabschnitt abgeschnitten werden. Hier sind Aortenbögen mit solchen Nähten bevorzugt, die Zwischenverriegelungen der Fäden aufweisen oder Teilnähte mit jeweiliger Endverriegelung besitzen.

Die maschinelle Fertigung kann auch noch dadurch erleichtert werden, dass mehrere Nähte, z.B. parallele Nähte oder Teilnähte, mit durchgehendem Faden genäht werden, der dann die Zwischenbereiche überbrückt, wie dies in Figur 6 schematisch dargestellt ist.

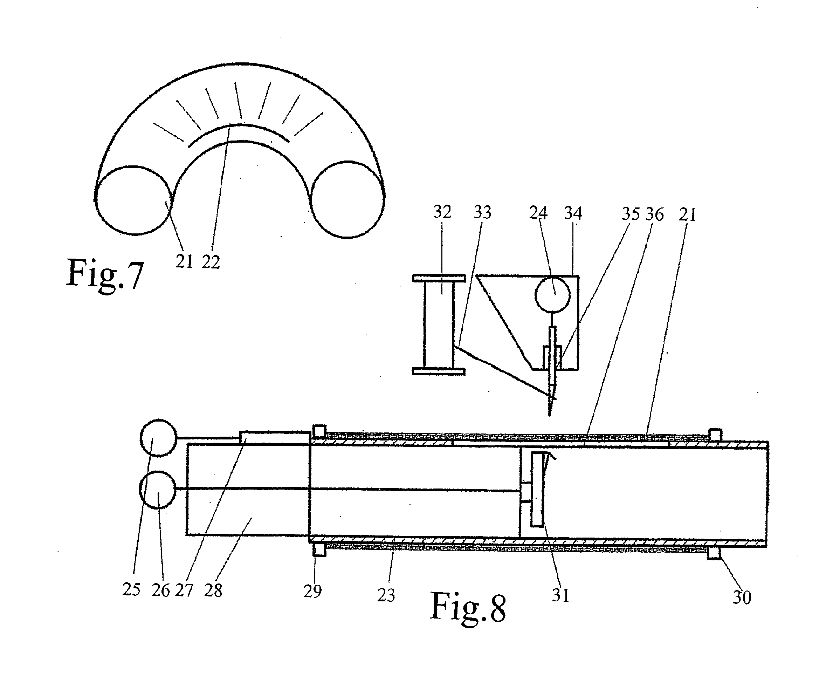

Fig. 7 stellt eine erfindungsgemäß hergestellte elastische Gefäßprothese dar. Sie besteht aus einem biokompatiblen, dauerfesten Material, beispielsweise aus Polyethylenterephthalat. Die Größenordnung der Durchmesser bewegt sich im zweistelligen Millimeterbereich, vorzugsweise im Bereich von 20 bis 40 mm. Während der Herstellung der Krümmung wird die Gefäßprothese gestaucht auf ein Stützrohr 23 aufgeschoben und mit wenigstens einer, sich wenigstens über einen Teil der Wandungslänge erstreckenden Naht 22 versehen. Diese sorgt nach dem Abziehen der Gefäßprothese 21 vom Stützrohr 23 und nach der Entspannung der elastischen Stauchung dafür, dass die Stauchung im Bereich der Naht 22 erhalten bleibt. Mit größer werdendem Abstand von der Naht 22 auf dem Umfang der Gefäßprothese 21 entspannt sich die Wandung immer deutlicher und kann sich wieder elastisch ausdehnen. Dadurch kommt es automatisch zu einer Verbiegung der Gefäßprothese 21. Die Naht 22 wird mit einem biokompatiblen Faden 33, z.B. aus Polyethylenterephthalat erzeugt. Es ist manchmal vorteilhaft, die Gefäßprothese 21 vor dem gestauchten Aufbringen auf das Stützrohr 23 umzuwenden, das heißt, die Außenseiten nach innen zu kehren und umgekehrt. Wendet man die Gefäßprothese 21 nach Fertigstellung wieder, sind die Verknotungen dann außen und stören nicht die Blutzirkulation.

Es sei noch erwähnt, dass der Schutzumfang des Verfahrens auch umfassen soll, eine bereits auf thermischem oder chemischem Wege erzeugte Vorkrümmung einer Gefäßprothese zu stabilisieren. Ferner umfasst das Verfahren bevorzugt den Einsatz einer Gefäßprothese , die eine Plissierung aufweist. Eine solche Gefäßprothese lässt sich besonders gleichmäßig kompaktieren bzw. stauchen.

## Patentansprüche

1. Verfahren zur Herstellung einer textilen Gefäßprothese (1) mit einer umlaufenden von Falten (4) gebildeten Plissierung und einer mindestens über einen Teilabschnitt ausgebildeten bogenförmigen Längsbiegung (2) durch asymmetrische verkürzende Raffung der Prothesenwand durch mindestens eine längs des Teilabschnittes verlaufende Längsnaht (7; 9; 10; 11), wobei die mindestens eine Längsnaht (7; 9; 10; 11) die Prothesenwand unter Erhalt der faltenbalgartigen Struktur (4) und in in Längsrichtung kompaktiertem Zustand der Plissierung (4) rafft,
**gekennzeichnet durch** die folgenden Schritte:
- Stauchung der textilen Gefäßprothese in axialer Richtung
- Versehen der Gefäßprothese mit wenigstens einer, sich wenigstens über einen Teil der Wandungslänge in axialer Richtung erstreckenden Naht, die mit Hilfe eines Fadens gebildet wird, und
- Entspannen der Gefäßprothese, wobei das Material im Bereich der Naht nicht in den ursprünglichen Zustand zurückfedert, wobei die Stauchung der Gefäßprothese auf einem Stützrohr vorgenommen wird und die Naht angebracht wird, solange sich die gestauchte Gefäßprothese auf dem Stützrohr befindet, und die genähte Gefäßprothese vom Stützrohr abgezogen wird.

2. Verfahren nach Anspruch 1, **dadurch gekennzeichnet, dass** das Stützrohr während des Nähvorgangs in axialer Richtung bewegt wird.

3. Verfahren nach Anspruch 1 oder 2, **dadurch gekennzeichnet, dass** eine Nähnadel durch die Gefäßprothese und ein in die Wandung des Stützrohres eingebrachtes Langloch in das Innere des Stützrohres eintaucht.

4. Verfahren nach einem der vorhergehenden Ansprüche, **dadurch gekennzeichnet, dass** der Faden durch einen Fadengreifer von der Nadel abgenommen und verkettet wird.

5. Verfahren nach Anspruch 4, **dadurch gekennzeichnet, dass** der Fadengreifer rotiert.

6. Verfahren nach Anspruch 4 oder 5, **dadurch gekennzeichnet, dass** das Stützrohr während seiner axialen Bewegung den rotierenden Fadengreifer umschließt.

7. Verfahren nach einem der vorhergehenden Ansprüche, **dadurch gekennzeichnet, dass** die Gefäßprothese vor der Stauchung vorzugsweise vor der Aufnahme auf das Stützrohr auf links gewendet wird, also die Innenoberfläche nach außen weist und umgekehrt.

8. Verfahren nach einem der vorhergehenden Ansprüche, **dadurch gekennzeichnet, dass** die Stauchung über eine Stauchungsvorrichtung vorgenommen wird.

9. Verfahren nach einem der vorhergehenden Ansprüche, **dadurch gekennzeichnet, dass** die gestauchte Gefäßprothese an wenigstens einem verstellbaren Anschlag abgestützt wird.

10. Verfahren nach einem der vorhergehenden Ansprüche, **dadurch gekennzeichnet, dass** eine Gefäßprothese mit einer umlaufenden Plissierung eingesetzt wird.

11. Verfahren nach einem der vorhergehenden Ansprüche, **dadurch gekennzeichnet, dass** eine Gefäßprothese mit einer thermisch oder chemisch vorgeformten Krümmung eingesetzt wird.

## Claims

1. A method of manufacture of a textile vascular prosthesis (1) with a circumferential pleating formed by folds (4), and with an arch-shaped longitudinal flexion (2) extending over at least a partial section and formed by asymmetric shortening gathering of the prosthesis wall by means of at least one longitudinal seam (7; 9; 10; 11) extending along the partial section; wherein the at least one longitudinal seam (7; 9; 10; 11) gathers the prosthesis wall while preserving the accordion-like structure (4) and the compacted state of the pleating (4) in the longitudinal direction,
**characterized by** the following steps:
- compression of the textile vascular prosthesis in the axial direction,
- providing the vascular prosthesis with at least one seam extending in the axial direction over at least a part of the wall length and formed using a thread, and
- releasing the vascular prosthesis, whereupon the material in the area of the seam does not spring back to the original state, wherein compression of the vascular prosthesis is effected on a support tube, and the seam is applied as long as the compressed vascular prosthesis is situated on the support tube, and the sewn vascular prosthesis is withdrawn from the support tube.

2. The method according to claim 1, **characterized in that** the support tube is moved in the axial direction during the sewing procedure.

3. The method according to claim 1 or 2, **characterized in that** a sewing needle passes through the vascular prosthesis and through an oblong hole formed in the wall of the support tube, into the interior of the support tube.

4. The method according to any one of the preceding claims, **characterized in that** the thread is taken up from the needle by a thread gripper and chain-stitched.

5. The method according to claim 4, **characterized in that** the thread gripper rotates.

6. The method according to claim 4 or 5, **characterized in that** the support tube, during its axial movement, encloses the rotating thread gripper.

7. The method according to any one of the preceding claims, **characterized in that** before being compressed, preferably before being received on the support tube, the vascular prosthesis is turned inside out, that is to say the inside surface faces outward, and vice versa.

8. The method according to any one of the preceding claims, **characterized in that** the compression is done using a compression device.

9. The method according to any one of the preceding claims, **characterized in that** the compressed vascular prosthesis is supported on at least one adjustable abutment.

10. The method according to any one of the preceding claims, **characterized in that** a vascular prosthesis with a circumferential pleating is used.

11. The method according to any one of the preceding claims, **characterized in that** a vascular prosthesis with a thermally or chemically preformed curvature is used.

## Revendications

1. Procédé de fabrication d'une prothèse vasculaire textile (1) avec un plissage continu formé par des plis (4) et avec une courbure longitudinale en forme d'arc (2) formée au moins sur une section partielle par un fronçage de raccourcissement asymétrique de la paroi de prothèse au moyen d'au moins une couture longitudinale (7; 9; 10; 11) s'étendant le long de la section partielle, dans lequel ladite au moins une couture longitudinale (7; 9; 10; 11) fronce la paroi de prothèse pour obtenir la structure en soufflet plissé (4) et dans l'état compacté en direction longitudinale du plissage (4),
**caractérisé par** les opérations suivantes:
- refouler la prothèse vasculaire textile en direction axiale;
- doter la prothèse vasculaire d'au moins une couture s'étendant au moins sur une partie de la longueur de la paroi en direction axiale, qui est formée à l'aide d'un fil, et
- détendre la prothèse vasculaire, dans lequel le matériau ne revient pas à son état initial dans la région de la couture, dans lequel on opère le refoulement de la prothèse vasculaire sur un tube de support et on applique la couture aussi longtemps que la prothèse vasculaire refoulée se trouve sur le tube de support, et on retire la prothèse vasculaire cousue du tube de support.

2. Procédé selon la revendication 1, **caractérisé en ce que** l'on déplace le tube de support en direction axiale pendant l'opération de couture.

3. Procédé selon la revendication 1 ou 2, **caractérisé en ce que** l'on plonge une aiguille à coudre à l'intérieur du tube de support à travers la prothèse vasculaire et un trou oblong pratiqué dans la paroi du tube de support.

4. Procédé selon l'une quelconque des revendications précédentes, **caractérisé en ce que** l'on retire le fil de l'aiguille et on l'enchaîne au moyen d'un dispositif de pinçage de fil.

5. Procédé selon la revendication 4, **caractérisé en ce que** le dispositif de pinçage de fil tourne.

6. Procédé selon la revendication 4 ou 5, **caractérisé en ce que** le tube de support entoure le dispositif de pinçage de fil tournant pendant son mouvement axial.

7. Procédé selon l'une quelconque des revendications précédentes, **caractérisé en ce que** la prothèse vasculaire est enroulée à gauche, donc la surface intérieure est orientée vers l'extérieur et inversement, avant le refoulement, de préférence avant le placement sur le tube de support.

8. Procédé selon l'une quelconque des revendications précédentes, **caractérisé en ce que** l'on opère le refoulement au moyen d'un dispositif de refoulement.

9. Procédé selon l'une quelconque des revendications précédentes, **caractérisé en ce que** l'on appuie la prothèse vasculaire refoulée sur au moins une butée réglable.

10. Procédé selon l'une quelconque des revendications précédentes, **caractérisé en ce que** l'on utilise une prothèse vasculaire avec un plissage continu.

11. Procédé selon l'une quelconque des revendications précédentes, **caractérisé en ce que** l'on utilise une prothèse vasculaire avec une courbure préformée par voie thermique ou chimique.
